# EUROPEAN PATENT APPLICATION

(11) **EP 2 402 446 A1**
(43) Date of publication of application: **04.01.2012**
(21) Application number: 10168039.5
(22) Date of filing: 30.06.2010
(51) Int. Cl.: C12N 15/29, C12N 15/82, C07K 14/415

(54) **Gene involved in the development of the seed**

(71) Applicant: Genoplante-Valor, 75015 Paris (FR); Universität Hamburg, 20146 Hamburg (DE)
(72) Inventor: The designation of the inventor has not yet been filed
(74) Representative: Flesselles, Bruno F.G.

(57) **Abstract**

The invention relates to the field of plant improvement, with the identification of the ZmPPR6 gene that is involved in development of the seed.

## Description

The invention relates to the field of plant improvement, with the identification of a gene that is involved in development of the seed.

In maize, the size, shape and content of the two seed compartments (embryo and endosperm) is determined during seed development, a process involving an estimated 1000 genes. Less than 10% are known today making the identification and characterization of the remaining genes a major scientific and agronomic challenge.

### BACKGROUND OF THE INVENTION

The seed of cereals is one of the most economically important and scientifically interesting structure in plant biology. It consists of the embryo and the endosperm, the two products of the double fertilization event. The endosperm originates from the central cells of the embryo sac, which are fertilized by one of the two haploid male gametes, and the embryo originates from the fusion of the second gamete with the oosphere.

In maize, the embryo consists of an embryonic axis surrounded by a single massive cotyledon, the scutellum. The embryonic axis is characterized by the presence of a root primordium with the root meristem at the basis, the scutellar node, the mesocotyl and the shoot primordium comprising of the apical meristem and four-five leaf initials. The endosperm functions as an embryonic annex that sustains the embryo during its development and its germination.

The endosperm is a complex tissue, observed in Angiosperm seeds. It is made from free-nuclear divisions, followed by cellularisation and the subsequent formation of a range of functional cellular domains.

As a nutritive tissue for the embryo during development of the seed, the endosperm is a storage organ in maize seeds; the endosperm also provides nutrients to the seedling on germination.

The endosperm consists in four domains:

The central area of the endosperm (first endosperm domain) consists of large cells with vacuoles, which store the reserves of starch and proteins (central starchy endosperm where genes involved in starch and in prolamin storage proteins biosynthesis are expressed), whilst the region surrounding the embryo (ESR that corresponds to the second endosperm domain) is distinguished by rather small cells, occupied for the major part by cytoplasm. The ESR may have a role in embryo nutrition or in establishing a physical barrier between the embryo and the endosperm during seed development.

The Basal Endosperm Transfer Layer (BETL that corresponds to the third endosperm domain) area is highly specialized to facilitate uptake of solutes during grain development. These transfer cells of the basal endosperm have specialised internal structures adapted to absorb solutes from the maternal pedicel tissue, and translocate these products to the developing endosperm and embryo. These transfer cells facilitate nutrient import into the maize kernel.

The fourth endosperm domain consists of the aleurone, which is the outer layer of the endosperm and accumulates proteins and oil.

The inventors here report a new gene involved in kernel development of the endosperm in plants. This gene encodes a PPR protein. PPR proteins are often required for the maturation of organellar RNA. Nevertheless, it has been reported that the ZmPPR protein is involved in seed development (WO 2007/057402).

Pentatricopeptide repeat (PPR) proteins form a large protein family that is particularly prevalent in land plants and includes 450 members in Arabidopsis thaliana (Lurin et al., 2004; O'Toole et al., 2008). The family members are defined by a tandem array of PPR motifs, each of which is a highly degenerate unit consisting of 35 amino acids and expected to fold into a pair of antiparallel helices (Small and Peeters, 2000). Most PPR proteins are predicted to localize to plastids or mitochondria (Lurin et al., 2004). PPR proteins are involved in almost all stages of gene expression, including transcription (Pfalz et al., 2006), splicing (Schmitz-Linneweber et al., 2006; de Longevialle et al., 2007, 2008), RNA cleavage (Hashimoto et al., 2003; Meierhoff et al., 2003; Hattori et al., 2007), RNA editing (Okuda et al., 2007; Chateigner-Boutin et al., 2008; Zhou et al., 2008), translation (Fisk et al., 1999; Williams and Barkan, 2003), and RNA stabilization (Yamazaki et al., 2004; Beick et al., 2008). The most probable explanation for these divergent roles is that they are sequence-specific RNA binding adaptors recruiting effector enzymes to the target RNA (Delannoy et al., 2007). Recent studies have shown that Arabidopsis PPR proteins play an essential, non-redundant role during embryogenesis (Cushing et al., 2005).

The mutation of the PPR gene identified in the present invention confers a severe reduction in endosperm development. Homozygous mutants have an affected plant growth.

The nucleic acid molecule characterized by the inventors is particularly useful for enhancing yield via over-expression in cells, tissues or organs that are limiting for yield. Its over-expression is also useful for increasing plant growth rate, grain filling, starch and proteins accumulations, speeding seed development.

### DESCRIPTION OF THE FIGURES

**Figure 1** describes a cob carrying mutant and wild type seeds in the left panel. In the middle panel, mutant (mu) and wild-type (wt) seeds are compared. The right panel represents homozygous mutant plants recovered by embryo rescue, compared to wild-type plants
**Figure 2** represents Arabidopsis thaliana plant: a 4-week old wild-type plant (left panel), a 10-week old mutant plant (middle panel) and a 4-week old plant complemented with the ZmPPR6 gene.
**Figure 3** is a schematic view of the pBIOS1700 plasmid.
**Figure 4** is a schematic view of the pBIOS1718 plasmid.
**Figure 5** is a schematic view of the pBIOS1701 plasmid.

### GENERAL DESCRIPTION

The present invention relates to an isolated nucleic acid molecule encoding a protein which alters the plant or endosperm development that comprises a sequence selected from the group consisting of:
a) a nucleotide sequence encoding a protein having an amino acid sequence as depicted in SEQ ID N° 2.
b) a nucleotide sequence as depicted in SEQ ID N° 1.

In another embodiment, the isolated nucleic acid encodes a protein which alters the plant or endosperm development that comprises a sequence selected from the group consisting of:
a) a nucleotide sequence encoding a protein having an amino acid sequence that presents at least 80 % sequence identity with SEQ ID N° 2;
b) a nucleotide sequence that presents at least 80 % sequence identity with SEQ ID N° 1 ;
c) a sequence hybridizing under stringent conditions with the complementary strand of a nucleic acid molecule as defined in (a) or (b).

Indeed, the disclosure of SEQ ID N° 2, SEQ ID N° 1 and their biological properties allows a person skilled in the art to identify variants of said protein and nucleic acid, which are usable in the same way of the disclosed biological material, are encompassed in the present invention and are thus equivalent in the context of the invention.

As used herein "variants" means that the sequence differs in one or more positions in comparison with the sequence SEQ ID N° 2 as long as it encodes a protein altering the plant or endosperm development, and possesses at least 70 % identity to the sequence SEQ ID N° 2, preferably at least 80 % identical, more preferably at least 85, 90, 91, 93, 95, 98, 99 or 99.9 % identical. Also preferably, the degree of identity is defined by comparison with the entire sequence of reference, SEQ ID N° 2. This percentage of identity has been obtained by the method of Needleman and Wunsch.

The disclosure of the present invention also makes it possible to identify nucleic acid sequences that are homologous to SEQ ID N° 1. Such a homologous nucleic acid sequence is at least 70 % identical to the sequence SEQ ID N° 1, preferably at least 80 %, 85% identical, more preferably at least 90, 91, 93, 95, 98, 90, 99.9 % identical. Also preferably, the degree of identity is defined by comparison with the entire sequence of reference, SEQ ID N° 1.

SEQ ID N° 3 represents a genomic sequence containing the intronless coding sequence of the ZmPPR6 protein represented by SEQ ID N° 2.

SEQ ID N° 4 and SEQ ID N° 5 and SEQ ID N° 6 represents orthologs of the ZmPPR6 protein.

Sequence comparisons between two (or more) polynucleotides or polypeptides are typically performed by comparing sequences of two optimally aligned sequences over a segment or "comparison window" to identify and compare local regions of sequence similarity. Optimal alignment of sequences for comparison may be conducted by the local homology algorithm of Smith and Waterman, Ad. App. Math 2: 482 (1981), by the homology alignment algorithm of Neddleman and Wunsch, J. Mol. Biol. 48: 443 (1970), by the search for similarity method of Pearson and Lipman, Proc. Natl. Acad. Sci. (U. S. A.) 85: 2444 (1988), by computerized implementation of these algorithms (GAP, BESTFIT, BLAST N, BLAST P, FASTA, and TFASTA in the Wisconsin Genetics Software Package, Genetics Computer Group (GCG), 575 Science Dr., Madison, WI), or by inspection.

Preferably, the percentage of identity of two polypeptides is obtained by using the Blast program (Blast 2 sequences), using the Blastp program and the BLOSUM62 matrix, with the following parameters: gap open: 11; gap extension: 1; x_dropoff: 0; expect: wordsize: 3; Filter: no. Alignment is performed on the full length of SEQ ID N° 2.

"Percentage of sequence identity" can also be determined by comparing two optimally aligned sequences over a comparison window, where the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

A preferred method uses the algorithm of Needleman and Wunsch.

Thus it is possible to identify such variant if the ZmPPR6 protein which differ, for example, by way of amino acid and/or nucleotide deletion(s), insertion(s), substitution(s), addition(s) and/or recombination(s) or any other modification(s) known in the art either alone or in combination from the above described amino acid sequences or their underlying nucleotide sequence(s). Methods for introducing such modifications in the nucleic acid molecules according to the invention are well-known to the person skilled in the art. The invention also relates to nucleic acid molecules the sequence of which differs from the nucleotide sequence of any of the above-described nucleic acid molecules due to the degeneracy of the genetic code.

The invention further relates to a protein encoded by said nucleic acid molecules. More specifically, the invention provides a protein that alters the plant or endosperm development encoded by a nucleic acid molecule as defined above. Preferably, a protein according to the invention may comprise, or consist in an amino acid sequence as depicted in SEQ ID N° 2.

The proteins encoded by the various variants of the above-described nucleic acid molecules share, or have close specific common characteristics, such as biological activity, molecular weight, conformation, etc., as well as physical properties, such as electrophoretic mobility, chromatographic behavior, sedimentation coefficients, pH optimum, temperature optimum, stability, solubility, spectroscopic properties, etc.

The person skilled in the art may also use the disclosure to identify nucleic acid molecules hybridizing under stringent conditions with the complementary strand of a nucleic acid molecule as defined above.

A nucleic acid molecule "hybridizes" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989).

Such an hybridizing nucleic acid alters the plant or endosperm development according to the invention and contains at least 15, 25, 35, 37, 50, 75, 100, 121 , 122, 127, 150, 200, 220, 240, 250, 270, 300, 350, 360, 365, 500, 750, 800, 900, 1000, 1100, 1200, 1300, 1400, 1500, 1600, 1700, 1800, 1850 nucleotides.

The parameters defining the stringency conditions depend on the temperature at which 50% of the paired strands separate (Tm).

For sequences comprising more than 30 bases, Tm is defined by the equation: Tm = 81.5 + 0.41 (%G+C) + 16.6 Log (concentration in cations) - 0.63 (%formamide) - (600/number of bases) (Sambrook et al., 1989). For sequences shorter than 30 bases, Tm is defined by the equation: Tm = 4(G+C) + 2(A+T).

Under appropriate stringency conditions, in which non-specific (aspecific) sequences do not hybridize, the temperature of hybridization is approximately between 5 and 30 °C, preferably between 5 and 10°C below Tm and hybridization buffers used are preferably solutions of higher ionic force like a solution 6*SSC for example.

The nucleotide sequences of the invention can be of artificial origin. It can be identified by screening of libraries of sequences by means of probes produced on the basis of SEQ ID N° 1. Such libraries are prepared by conventional techniques of molecular biology, known to persons skilled in the art. The nucleotide sequences according to the invention can also be prepared by chemical synthesis, or by mixed methods including the chemical or enzymatic modification of sequences obtained by screening banks.

According to an embodiment of the invention, a nucleic acid molecule which alters the plant or endosperm development consists in SEQ ID N° 1.

The nucleic acid molecules according to the invention and equivalents thereof can be isolated from various plant species, notably angiosperm plants, monocotyledons or dicotyledons and are preferably nucleic acid molecules isolated from a cereal or an oily plant. Still preferably, the nucleic acid molecules are isolated from a plant selected from the group consisting of maize, rice, sorghum, wheat, barley, rye, *Brassica napus,* pea, sunflower and sugar cane. Still preferably, the plant is maize.

It is possible for the person skilled in the art to isolate with the help of the ZmPPR6 nucleotide sequence of the invention, corresponding genes from other species.

This can be done by conventional techniques known in the art, for example, by using a sequence as depicted in SEQ ID N° 1 as a hybridization probe or by designing appropriate PCR primers. It is possible to start with coding DNA sequences or Protein sequences via TBLASTN queries. The approach used to isolate rice orthologs of the ZmPPR6 gene, for example, is to use the Protein sequence of ZmPPR6, do a tblastn with this sequence against Rice ESTs, then use this EST to find the genomic sequence or directly use tblastn against the rice genome sequence. The same approach is used to isolate orthologs of the ZmPPR6 gene in other species.

The invention also relates to a protein encoded by a nucleic acid molecule according to the invention. Preferably, the protein consists of an amino acid sequence as depicted in SEQ ID N° 2, or being at least 70 % identical to SEQ ID N° 2, preferably at least 85% identical, more preferably at least 90, 91, 95, 98, 90, 99.9 % identical to SEQ ID N° 2, calculated as described above.

Another object of the present invention is a nucleotide construction, referred to as an expression cassette comprising a ZmPPR6 coding nucleic acid molecule as defined above operatively linked to regulatory elements allowing the expression in prokaryotic and/or eukaryotic host cells.

"Operatively linked' refers to functional linkage between a ZmPPR6 nucleic acid molecule (altering the plant or endosperm development) according to the invention and a promoter sequence (regulatory element having a promoter activity). The ZmPPR6 nucleic acid molecule can be placed in the sense or antisense orientation.

The promoter sequence can be of a heterologous origin. Any suitable promoter could be used. It could be a constitutive promoter. It could also be for example a tissue-specific promoter such as a seed-specific or a BETL-specific promoter. Numerous tissue-specific promoters are described in the literature and any one of them can be used.

Among constitutive promoters, one can use for example the CsVMV promoter (Verdaguer et al, 1996), the rice actin promoter (McElroy et al., 1990), the CAMV 35 S or the 19S promoter (Kay et al., 1987).

Preferably, the promoter used allows expression in the endosperm. More preferably, the promoter used allows expression in the BETL and still more preferably, the promoter is BETL-specific.

Advantageously, promoters are for example pMRP1 which is expressed in the central cell and in the BETL region prior to and after BETL cellularisation (Gomez et al (2002), pMEG1 (Gutierrez-Marcos et al 2004), pBETL1 (Hueros et al 1999) and pBETL2 (WO 99/50427) which are BETL-specific promoters.

"BETL-specific promoter" means, as used in the present invention, that the promoter has a predominant pattern expression in the BETL, and preferably an exclusive pattern expression in the BETL.

The said ZmPPR6 nucleic acid molecule can also be associated with other regulating elements such as transcription termination sequences (terminators). By way of examples of such sequences, it is possible to cite the polyA 35S terminator of the cauliflower mosaic virus (CaMV), described in the article of Franck et al. (1980) and the NOS terminator corresponding to the region in the non-coding 3' region of the nopaline synthase gene of the Ti-plasmid of the Agrobacterium tumefaciens nopaline strain (Depicker et al. 1992). Preferably, the terminator used is the Nos terminator.

According to the invention, the expression cassette, comprising an ZmPPR6 nucleic acid molecule as defined above, operatively linked to regulatory elements allowing the expression in prokaryotic and/or eukaryotic host cells (such as a promoter sequence) may further comprise one or several selection marker genes for plants, useful for transformation and selection.

In the present invention, the term "selectable marker", "selectable gene", "selectable marker gene", "selection marker gene", "marker gene" are used interchangeably.

These selectable markers include, but are not limited to, antibiotic resistance genes, herbicide resistance genes or visible marker genes. Other phenotypic markers are known in the art and may be used in this invention.

A number of selective agents and resistance genes are known in the art. Notably the selectable marker used can be the bar gene conferring resistance to bialaphos (White et al., 1990), the sulfonamide herbicide Asulam resistance gene, sul (described in WO 98/49316) encoding a type I dihydropterate synthase (DHPS), the nptll gene conferring resistance to a group of antibiotics including kanamycin, G418, paromomycin and neomycin (Bevan et al., 1983), the hph gene conferring resistance to hygromycin (Gritz et al., 1983), the EPSPS gene conferring tolerance to glyphosate (US 5,188,642), the HPPD gene conferring resistance to isoxazoles (WO 96/38567), the gene encoding for the GUS enzyme, the green fluorescent protein (GFP), expression of which, confers a recognisable physical characteristic to transformed cells, the chloramphenicol transferase gene, expression of which, detoxifies chloramphenicol.

Advantageously, the selectable marker gene is inserted between a promoter and a terminator in a second expression cassette. Said second expression cassette is integrated in the same vector as the expression cassette containing the ZmPPR6 nucleic acid molecule under transcriptional control of a promoter according to the invention.

According to this advantageous embodiment, the marker gene is preferably controlled by a promoter which allows expression in cells, thus allowing selection of cells or tissue containing the marker at any stage of development of the plant. Preferred promoters are the promoter of nopaline synthase gene of Agrobacterium, the promoter derived from the gene which encodes the 35S subunit of cauliflower mosaic virus (CaMV) coat protein, and the rice actin promoter. However, any other suitable second promoter may be used.

Any terminator may be used. Preferred terminators are the 3'CaMV and Nos terminator as previously described.

Advantageously, the expression cassette containing the selectable marker gene is comprised between two Ds elements (transposons) in order for its removal at a later stage by interacting with the Ac transposase. This elimination system is described in Yoder et al. (1993).

In another embodiment, for the transformation step, two vectors could be used, the first one comprising the expression cassette containing the ZmPPR6 nucleic acid molecule and the second one comprising the expression cassette containing the selectable marker gene. The same host cell being transformed with these two vectors (co- transformation).

The expression cassettes according to the invention may additionally contain transit peptide sequences. There are numerous examples in the art of transit peptides which may be used to deliver a target protein into a plastid organelle such as the small subunit (SSU) transit peptide of ribulose biphosphate carboxylase.

Other elements like introns and enhancers can also be present in the nucleic sequence of interest in order to improve the expression of the gene of interest. Among useful introns, the first intron of maize adh1 S can be placed between the promoter and the coding sequence. This intron when included in a gene construct increased the expression of the desired protein in maize cells. One also can use the 1 st intron of the shrunken 1 gene of the maize (Maas et al., 1991), the 1 st intron of the catalase gene of the bean catalase (CAT-1 ) (Ohta et al., 1990), the 2nd intron of the ST-LS1 gene of potato (Vancanneyt et al. 1990), the DSV intron of the yellow dwarf virus of tobacco (Morris et al., 1992), the actin-1 intron (act-1 ) of rice (McElroy et al., 1990), FAD 2 intron (WO 2006/003186) and intron 1 of thosephosphate isomerase (TPI) (Snowdon et al., 1996).

Preferentially, the intron used in the present invention is the Sh1 intron.

The expression cassettes may additionally contain 5' leader sequences. Such leader sequences can act to enhance translation. Such 5' leader sequences are known in the art and include, but are not limited to, picornavirus leaders, for example, the EMCV leader (Encephalomyocarditis 5' noncoding region) (Elroy-Stein, Fuerest, and Moss B., 1989) ; potyvirus leaders, for example, the TEV leader (Tobacco etch Virus) (Allison et al., 1986) ; the human immunoglobulin heavy-chain binding protein leader (BiP) (Macejack and Sarnow, 1991) ; the untranslated leader from the coat protein mRNA of alfalfa mosaic virus (AMV RNA 4) (Jobling and Gehrke, 1987) ; the tobacco mosaic virus leader (TMV) (Gallie et al., 1989) ; and the maize chlorotic mottle virus leader (MCMV) (Lommel et al., 1991). Other methods known to enhance translation can be utilized, for example introns, and the like.

In preparing the expression cassettes, the various DNA sequences or fragments may be manipulated, so as to provide DNA sequences or fragments in the proper orientation and, as appropriate, in the proper reading frame. Towards this end, adapters or linkers may be employed to join the DNA fragments and/or other manipulations may be required to provide convenient restriction sites, removal of superfluous DNA, removal of restriction sites, or the like. For this purpose, in vitro mutagenesis, primer repair, restriction, annealing, ligation, PCR, or the like may be employed, where nucleotide insertions, deletions or substitutions, for example transitions and transversions, may be involved. These techniques are well known by those skilled in the art.

Another object of the invention is any nucleotide vector referred to as an expression vector, such as a plasmid, which can be used for transforming host cells, characterized in that it contains at least an expression cassette as defined above. The construction of expression vectors for the transformation is within the capability of one skilled in the art following standard techniques.

The decision as to whether to use a vector, or which vector to use, is guided by the method of transformation selected, and by the host cell selected.

Where a naked nucleic acid introduction method is used, then the vector can be the minimal nucleic acid sequences necessary to confer the desired phenotype, without the need for additional sequences.

Possible vectors include the Ti plasmid vectors, shuttle vectors designed merely to maximally yield high numbers of copies, episomal vectors containing minimal sequences nec essary for ultimate replication once transformation has occurred, transposon vectors, including the possibility of RNA forms of the gene sequences. The selection of vectors and methods to construct them are commonly known to persons of ordinary skill in the art and are described in general technical references (Mullis, KB (1987), Methods in Enzymology).

For other transformation methods requiring a vector, selection of an appropriate vector is relatively simple, as the constraints are minimal. The apparent minimal traits of the vector are that the desired nucleic acid sequence be introduced in a relatively intact state. Thus, any vector which produces a plant carrying the introduced DNA sequence should be sufficient. Also, any vector which introduces a substantially intact RNA which can ultimately be converted into a stably maintained DNA sequence should be acceptable.

However, any additional attached vector sequences which confer resistance to degradation of the nucleic acid fragment to be introduced, which assists in the process of genomic integration or provides a means to easily select for those cells or plants which are actually, in fact, transformed are advantageous and greatly decrease the difficulty of selecting useable transgenic plants.

The vector can exist, for example, in the form of a phage, a plasmid or a cosmid. The construction of such expression vectors for transformation is well known in the art and uses standard techniques. Mention may be made of the methods described by Sambrook et al. (1989).

Another object of the invention is a host cell, containing at least an expression vector as described above.

The decision as to whether to use a host cell, or which host cell to use, is guided by the method of transformation.

The host cell can be any prokaryotic or eukaryotic cell. Any of a large number of available and well-known host cells may be used in the practice of this invention. The selection of a particular host is dependent upon a number of factors recognized by the art. These include, for example, compatibility with the chosen expression vector, bio-safety and costs. Useful hosts include bacteria such as *E. coli* sp. or *Agrobacterium.* A plant host cell, may be also used, notably an angiosperm plant cell, monocotyledon or dicotyledon plant cell, particularly a cereal or oily plant cell, and more particularly selected from the group consisting of maize, wheat, barley, rice, rape, *Brassica napus,* sugar cane, sorghum, pea and sunflower. Still preferably the plant host cell is from maize.

More particularly, the host cell used in carrying out the invention is *Agrobacterium tumefaciens,* according to the method described in the article of An et al., 1986, or *Agrobacterium rhizogenes,* according to the method described in the article of Jouanin et al., 1987.

The invention also concerns a transgenic plant, or a part of a transgenic plant, comprising stably integrated into its genome, as a transgene, a nucleic acid molecule according to the present invention operatively linked to regulatory elements allowing transcription and/or expression of the nucleic acid molecule in plant cells. "Part of a transgenic plant", according to the present invention, means in particular fruit, seed, grain, or pollen.

The invention also concerns a transgenic plant, or a part of a transgenic plant, comprising such a host cell or generated from such a host cell.

Where the plant contains endogenously a ZmPPR6 gene according to the invention, it will be understood that the transgenic plant according to the invention comprises an additional "exogenous" ZmPPR6 gene, for instance integrated by transgenesis.

A whole plant can be regenerated from a single transformed plant cell. Thus, in a further aspect the present invention provides transgenic plants (or parts of them) including nucleic acid molecules in accordance with the invention. The regeneration can proceed by known methods.

The seeds, which grow by fertilization from this plant, also contain this transgene in their genome.

Advantageously, the transgenic plant obtained can produce grains with a larger (bigger) endosperm (increased endosperm size) in comparison with a non-transformed plant. In addition to increases in yield such transgenic plants may have grains with modified starch, oil or protein contents. In particular, said modification in starch, oil, or protein contents consists in an increase of starch, oil, or protein accumulation.

A plant or part of a plant according to the invention could be a plant or a part of it from various species, notably an angiosperm, monocotyledon or dicotyledon, preferably a cereal or oily plant, and more preferably selected from the group consisting of maize, rice, sorghum, wheat, barley, rape, *Brassica napus,* sugar cane, and sunflower. Still preferably, the plant is maize.

As used herein, the term "oily plant" denotes a plant that is capable of producing oil, and preferably that is cultivated for oil production, such as sunflower or a plant of *Brassica* species.

Hybrid plants obtained by crossing plants according to the invention also form part of the invention.

Transgenic plants are obtained by transformation of plants with expression vectors containing the ZmPPR6 gene as a transgene, using any one of the techniques known to one skilled in the art.

It is possible to cite in particular the methods of direct transfer of genes such as direct micro-injection into plant embryoids, vacuum infiltration, electroporation, direct precipitation by means of PEG or bombardment by gun of particules covered with the plasmidic DNA of interest. References for these methods may be found in WO 2007/057402.

It is also possible to infect the plant with a bacterial strain, in particular *Agrobacterium.* According to one embodiment of the method of the invention, the vegetable cells are transformed by a vector according to the invention, the said cell host being able to infect the said vegetable cells by allowing the integration, in the genome of the latter, of the nucleotide sequences of interest initially contained in the above-mentioned vector genome. Advantageously, the above-mentioned cell host used is *Agrobacterium tumefaciens* or *Agrobacterium rhizogenes.*

For example, the transformation of vegetable cells can be achieved by the transfer of the T region of the tumour-inducing extra-chromosome circular plasmid of *Agrobacterium tumefaciens,* using a binary system. To do this, two vectors are constructed. In one of these vectors the T region has been eliminated by deletion, with exception of the right and left borders, a marker gene being inserted between them to allow selection in the plant cells. The other partner of the binary system is an auxiliary plasmid Ti, a modified plasmid which no longer has any T region but still contains the virulence genes vir necessary to the transformation of the vegetable cell.

According to a preferred mode, it is possible to use the method described by Ishida et al. (1996) for the transformation of monocotyledons.

The engineered plant material may be selected or screened for transformants (those that have incorporated or integrated the introduced nucleotide construction(s)). Such selection and screening methodologies are well known to those skilled in the art. The selection and screening method is chosen depending on the marker gene used. An isolated transformant may then be regenerated into a plant.

Normally, regeneration is involved in obtaining a whole plant from the transformation process. The term "regeneration" as used herein, means growing a whole plant cell, a group of plant cells, a plant part or a plant piece (for example, from a protoplast, callus, or tissue part).

Methods of regenerating whole plants from plant cells are known in the art, and the method of obtaining transformed and regenerated plants is not critical to this invention.

In general, transformed plant cells are cultured in an appropriate medium, which may contain selective agents such as antibiotics, where selectable markers are used to facilitate identification, of transformed plant cells. Once callus forms, shoot formation can be encouraged by employing appropriate plant hormones in accordance with known methods and shoots transferred to rooting medium for regeneration of plants. The plants may then be used to establish repetitive generations, either from seeds or using vegetative propagation techniques.

The invention also concerns the use of the transgenic plants obtained according to the invention, or parts of these plants, in particular seeds, grains, and fruits for preparing derived products, in particular food products.

The products obtained, whether it be seeds with a higher oil content, flours of seeds or grains with a higher starch, protein or oil content, also come within the scope of the invention, The invention also provides any composition for human or animal food prepared from the said obtained products.

In particular, the nucleic acid of the invention may be used in a method for obtaining a plant having increased seed size, said method comprising the steps consisting of:
a) transforming at least one plant cell or plant tissue by means of at least a vector as defined previously;
b) cultivating the cell(s) or plant tissue thus transformed so as to generate a plant containing in its genome at least an expression cassette according to the invention, whereby said plant has increased seed size.

According to the invention, "increased seed size" means that the transformed plant have a seed bigger (larger) than a seed from a wild type plant (non-transformed plant). Such an increase in seed size is desirable to increase seed and or endosperm yield and to improve the germination and vigour of seedlings.

The invention also relates to a method for increasing plant growth rate, said method comprising the steps consisting of:
a) transforming at least a plant cell or plant tissue by means of at least a vector as defined previously; b) cultivating the cell(s) or plant tissue thus transformed so as to generate a plant containing in its genome at least an expression cassette according to the invention.

Plant growth rate can be increased (as compared with the growth rate of a non-transgenic plant) with either an improvement in final yield or earlier flowering and harvest.

It may also be possible to obtain a plant having decreased seed moisture content, (as compared to a non-transgenic plant) said method comprising the steps consisting of
a) transforming at least a plant cell or plant tissue by means of at least a vector as defined previously;
b) cultivating the cell(s) or plant tissue thus transformed so as to generate a plant containing in its genome at least an expression cassette according to the invention, whereby said plant has decreased seed moisture content.

It is indeed postulated that over-expression of the ZmPPR6 protein into the endosperm (with the aim of an endosperm promoter) shall increase the speed of the seed development leading to a seed having a lower moisture content than a normal seed (wild type) at harvest. This is of interest and useful for reducing drying costs.

The invention also relates to a method for increasing plant starch and proteins accumulation (as compared to a non-transgenic plant), said method comprising the steps consisting of:
a) transforming at least a plant cell or plant tissue by means of at least a vector as defined previously;
b) cultivating the cell(s) or plant tissue thus transformed so as to generate a plant containing in its genome at least an expression cassette according to the invention, whereby said plant has increased starch and proteins content.

The invention also relates to a method for increasing grain filling (as compared to a non-transgenic plant), said method comprising the steps consisting of:
a) transforming at least a plant cell or plant tissue by means of at least a vector as defined previously;
b) cultivating the cell(s) or plant tissue thus transformed so as to generate a plant containing in its genome at least an expression cassette according to the invention.

The invention also relates to a method for speeding seed development (as compared to a non-transgenic plant), said method comprising the steps consisting of:
a) transforming at least a plant cell or plant tissue by means of at least a vector as defined previously;
b) cultivating the cell(s) or plant tissue thus transformed so as to generate a plant containing in its genome at least an expression cassette according to the invention.

The present invention will be further understood in view of the figures and following examples.

### Example 1: Characterization of mutants in the ZmPPR6 gene

The ORF of ZmPPR6 containing 1467 bp (SEQ ID N°2) encodes a pentatricopeptide repeat protein of 489 amino acid residues corresponding to a predicted molecular weight of 55,5 kDa (SEQ ID N° 1).

EST homology search in the NCBI DB provided 8 unpublished cDNA hits (3 from maize CD440816.1 EE027064.2 CD950140.1, 3 from sugarcane CA201270.1 CA089494.1 CA089528.1 and 2 from wheat BJ319078.1 BJ313327.1). A 2460 bp genomic clone (MAGIv4_67802) resulted from a blast search in the MAGI DB revealed the full length of the intronless ZmPPR6 and is represented by SEQ ID N° 3.

Protein blast search showed high homology to an unpublished Arabidopsis PPR protein (AtPPR) indicating that it might be orthologous.

10 sequently grouped PPR domains were predicted by ScanProsite in both, maize and Arabidopsis protein. TargetP 1.1 Server-prediction results revealed a putative mitochondrial subcellular localisation signal, which was also predicted for the Arabidopsis homologous PPR protein.

Three independent mutants presenting a mutator transposon insertion in the gene cording for the ZmPPR6 protein were isolated.

H2352, which present an insertion in the 5' UTR (at nucleotide - 29 before the ATG start codon)

J0487, which presents an insertion at 394 in the CDS of the gene, so that the corresponding protein contains only the first 131 amino acids of SEQ ID N° 2.

D0432 , which presents an insertion at 793 in the CDS of the gene, so that the corresponding protein contains only the first 264 amino acids of SEQ ID N° 2.

Follow-up of Mu insertions in the PPR gene was performed using a PCR-based method, which uses an efficient transposon terminal inverted repeat (TIR)-specific primer (OmuA, CTTCGTCCATAATGGCAATTATCTC SEQ ID N° 7 and ppr6-gene specific primers designed in the first half of the gene (antisense: PPR_R03 ACAATGTCAGGCTGGCAACC SEQ ID N° 8; sense PPR_F03 GAAATGGGTGGCTTCCACTTC, SEQ ID N° 9).

Samples were taken from immature ears at 13 days after pollination (DAP), which is the earliest developmental stage at which wildtype and mutant kernels can be visually, and unambiguously, distinguished in all the lines.

### Example 2: Tissue specific expression of ZmPPR6

ZmPPR6 spatial expression was determined by a semiquantitative RT-PCR. The main expression of ZmPPR6 was observed during kernel development (4-21 dap). Expression was stronger in embryo than in endosperm. A low expression was found in non kernel tissues. ZmPPR6 mRNA could not be detected by conventional Northern blot hybridization, indicating that it is expressed at very low level.

### Example 3: ZmPPR6 mutant phenotype

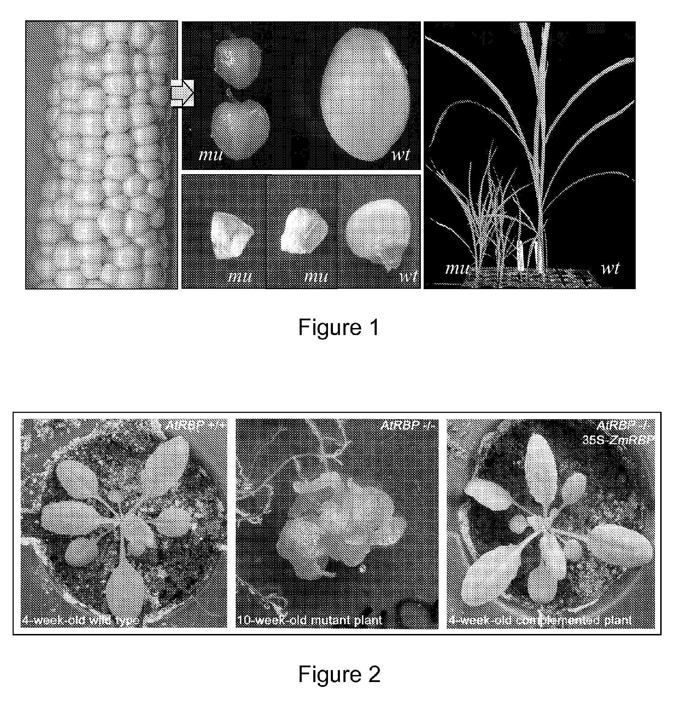

ZmPPR6 mutants are macroscopically recognizable as early as 10 DAP, because of the pale and much smaller appearance of mutant caryopses. At 20 DAP, ZmPPR6-/- embryos are much smaller than wildtype sibling embryos (Figure 1).

The mutation seems to affect kernel development, growth and endosperm filling. Dried mutant kernels are not able to germinate on soil or MS medium. Homozygous mutant plants recovered by embryo rescue exhibited a dwarf-like phenotype as they did not exceed a size of approximately 30 cm. Furthermore, a significant alteration in their vegetative architecture regarding floral abnormality was observed: mutant plants were not able to generate tassels (male sterility) instead of that a terminal positioned ear (female flower) was observed.

Closer examinations of cellular alterations by comparing wt and mu 12 DAP kernel sections provided a significant reduction of the starch amount in the endosperm of mu kernels.

### Example 4: AtPPR-KO shows ZmPPR6-related seed phenotype

The Arabidopsis genome contains only one close ZmPPR6 homologous gene which was named AtPPR. Phenotype and molecular analysis of three Arabidopsis T-DNA knock out lines (obtained from the NASC stock centre) was carried out.

A ZmPPR6-related seed phenotype was observed in all three lines. One quarter of the seeds within a mature silique appeared translucent, while the remaining three-quarters were wildtype. Closer examination of the seed phenotype of the three lines revealed a retarded embryo development. Dried mutant seeds never germinate on soil. A small fraction of these seeds could be recovered on MS medium showing an abnormal phenotype and obviously delayed development.

### Example 5: AtPPR mutant complementation by ZmPPR6 expression

Heterozygous AtPPR-KO plants were transformed with a T-DNA construct contaning ZmPPR6 under the transcriptional control of the 35S promoter. In the T1-generation after basta treatment a homozygous AtPPR-KO plant containing the ZmPPR6 construct could be identified by genomic and RT-PCR.

Complementation by ZmPPR6 of the homozygous AtPPR-KO mutant restored the wildtype phenotype. Furthermore, germination and growth of complemented AtPPR-KO plant were indistinguishable from the wildtype (Figure 2). This genetic complementation assay thus confirmed that the AtPPR mutant phenotype was indeed caused by the T-DNA insertion mutation in the AtPPR gene.

### Example 6: ZmPPR6 is localized in mitochondria

The subcellular localization of ZmPPR6 was investigated by transient biolistic co-transformation of Black Mexican Sweet cells. ZmPPR6 was translational fused with dsRed. ZmEmp4, a PPR protein known to be localized in mitochondria was fused with GFP and used as reference. Overlapping of both fluorescent signals in the merged image confirmed the prediction of the mitochondrial subcellular localization of ZmPPR6.

### Example 7: interaction of PPR with other proteins

In a co-immunoprecipitation approach, the 5' UTR of mitochondrial rps3 was identified as a potential target RNA of ZmPPR6. To examine the expression of rps3, the co- immunoprecipitated 5' UTR of rps3 is currently used as a probe in a Northern blot hybridization with total RNA from wt and mu embryos. EMSA (electrophoretic moblility shift assay) for confirming interaction between ZmPPR6 and 5' UTR of rps3 is performed.

### Exemple 8: generation of plants surexpressing ZmPPR6

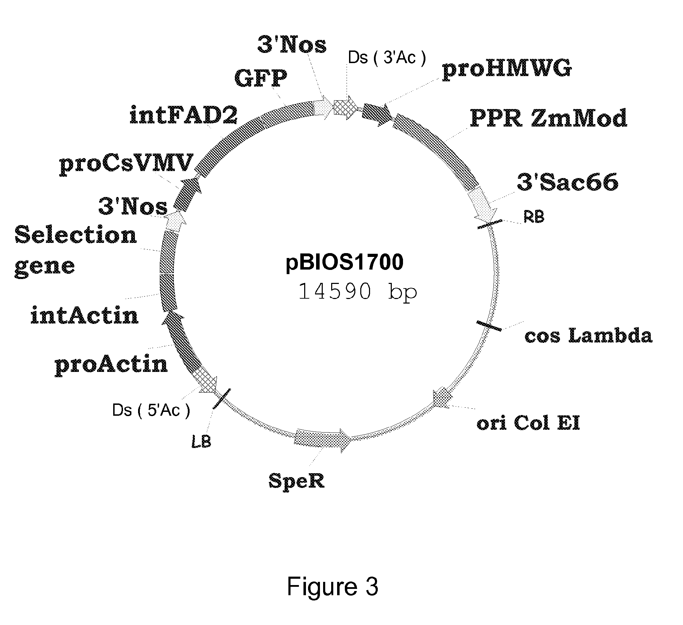

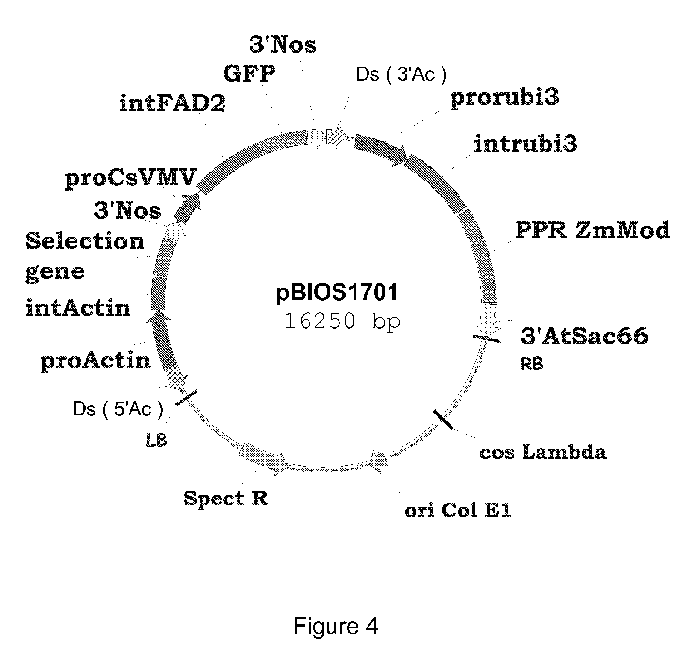

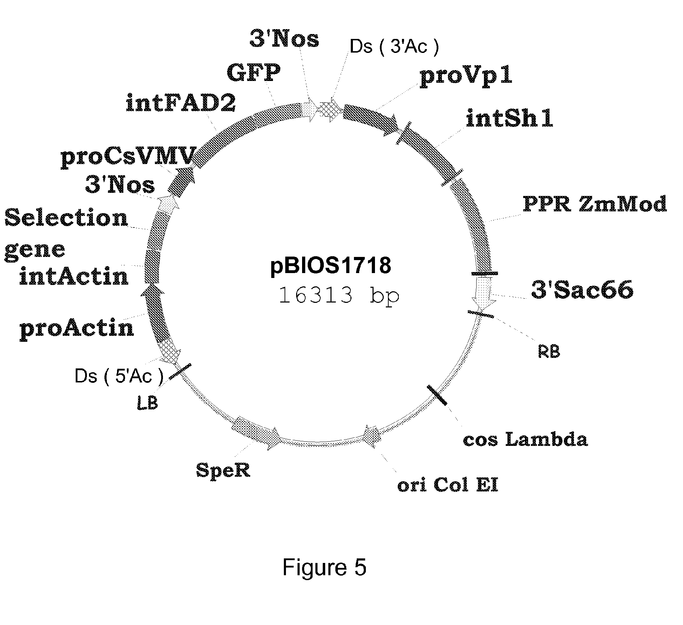

Four promoters were chosen for the transgenic constructs in order to overexpress ZmPPR6:
- pHMWG, linked to the actin intron : expression in the albumen; contruct pBIOS1700 (Figure 3).
- pVP1, linked to the shrinken1 intron : expression in embryon + aleurone (since RT-PCR experiment showed that the PPR cDNA is highly expressed in the embryo); construct pBIOS1718 (Figure 4).
- rice ubiquitin promoter, linked to the rice ubiquitin intron: ubiquitous expression; construct pBIOS1701 (Figure 5).
- maize Ubiquitin promoter was used and a strep tag was linked at the 5' of the ZmPPR6 sequence.

Analysis of T1 generation of transgenic maize plants expressing ZmPPR6 under the control of these promoters is in progress.

### REFERENCES

Allison et al. (1986), Virology, 154 :9-20
An et al. (1986), Plant Physiology, 81 :86-91.
Beick et al. Mol Cell Biol. 2008 Sep;28(17):5337-47. Epub 2008 Jun 30.
Bevan et al. (1983), Nature, 304 :184-187.
Chateigner-Boutin et al. Plant J. 2008 Nov;56(4):590-602. Epub 2008 Aug 23.
Cushing et al. Planta. 2005 Jun;221 (3):424-36. Epub 2005 Jan 13.
de Longevialle et al. Plant J. 2008 Oct;56(1):157-68. Epub 2008 Jun 28.
de Longevialle et al. Plant Cell. 2007 Oct;19(10):3256-65. Epub 2007 Oct 26.
Delannoy et al. Biochem Soc Trans. 2007 Dec;35(Pt 6):1643-7. Review.
Depicker et al. (1992), Mol. Gen. Genet, 235(2-3) :389-396.
Elroy-Stein et al. (1989) Proc Natl Acad Sci U S A. Aug;86(16) :6126-30.
Fisk et al. (1999) EMBO J. 18, 2621-2630
Franck et al. (1980), Cell, 21 (1 ) :285-94.
Gallie, D. R. et al. (1989), Molecular Biology of RNA, pages 237-256.
Gomez et al. (2002), Plant Cell, 14, 599-610.
Gritz et al. (1883) Gene. Nov; 25(2-3):179-88.
Gutierrez-Marcos JF, et al. (2004), Plant Cell. 16, 1288-301.
Hashimoto et al. Plant J. 2003 Nov;36(4):541-9
Hattori et al. J Biol Chem. 2007 Apr 6;282(14):10773-82. Epub 2007 Feb 5.
Hueros, G. et al. (1999), Plant Physiol. 121 , 1 143-1 152.
Ishida Y. et al. (1996), Nature Biotechnol. 14, 745-50.
Jenkins E. et al. (1999) Plant Cell Environ 22: 159-167.
Jobling, S.A., and Gehrke, L (1987), Nature, 325 :622-625.
Jouanin et al. (1987), Plant Science, 53 :53-63.
Kay et al. (1987), Science, 236 :1299-1302.
Lommel, S.A. et al. (1991 ), Virology, 81 :382-385.
Lurin et al. (2004), Plant Cell. Aug;16(8):2089-103.
Maas et al. (1991 ), Plant Molecular Biology, 16 :199.
Macejack, D. G., and P. Sarnow (1991 ), Nature, 353 :90-94.
McElroy et al. (1990), Plant Cell, 2 :163-171.
Meierhoff et al. Plant Cell. 2003 Jun;15(6):1480-95.
Morris et al. (1992), Virology, 187 :633
Mullis, KB (1987), Methods in Enzymology\ 55 :335.
O'Toole et al. Mol Biol Evol. 2008 Jun;25(6):1120-8. Epub 2008 Mar 14.
Ohta et al. (1990), Plant Cell Physiology, 31 :805.
Okuda et al. Proc Natl Acad Sci U S A. 2007 May 8;104(19):8178-83. Epub 2007 May 2.
Pfalz et al. Plant Cell. 2006 Jan;18(1):176-97. Epub 2005 Dec 2.
Sambrook et al. (1989), Molecular Cloning, A Laboratory Manual, Cold Spring Harbour Laboratory Press p. 9.54-9.62. Scanlon, M.J. et al. (1994) Genetics 136, 281 -294.
Schmitz-Linneweber et al. Plant Cell. 2006 Oct;18(10):2650-63. Epub 2006 Oct 13.
Small and Peeters Trends Biochem Sci. 2000 Feb;25(2):46-7
Snowdon et al. (1996), Plant Molecular Biology, 31 :689.
Vancanneyt et al. (1990) Mol. Gen. Genet. Jan;220(2):245-50.
Verdaguer B. et al. (1996) Plant Mol. Biol. 31 , 1 129-39.
White, J. et al. (1990) Nucl. Acid. Res. 18, 1062.
Yoder et al., (1993) Biotechnology, 12, 263-292.
Williams PM, Barkan A. Plant J. 2003 Dec;36(5):675-86.
Yamazaki et al. Plant J. 2004 Apr;38(1):152-63.
Zhou et al. Plant J. 2008 Dec 2.
WO 99/50427
WO 2007/057402
WO 98/49316
US 5,188,642
WO 96/38567
WO 2006/003186

## Claims

1. An isolated nucleic acid molecule encoding a protein which alters the plant or endosperm development that comprises a sequence selected from the group consisting of:
a) a nucleotide sequence encoding a protein having an amino acid sequence as depicted in SEQ ID N° 2;
b) a nucleotide sequence as depicted in SEQ ID N° 1.

2. An expression cassette comprising a nucleic acid molecule according to claim 1 operatively linked to regulatory elements allowing the expression in prokaryotic and/or eukaryotic host cells.

3. The expression cassette according to claim 2 wherein the promoter allows expression in the endosperm.

4. The expression cassette according to anyone of claim 2 or 3 wherein the promoter is a BETL-specific promoter.

5. The expression cassette according to anyone of Claims 2 to 4 which further comprises a selection marker gene for plants.

6. An expression vector containing at least an expression cassette according to any one of Claims 2 to 5.

7. A host cell containing at least a vector according to Claim 7.

8. A transgenic plant, or a part of a transgenic plant, comprising, as a stably integrated transgene into its genome, a nucleic acid molecule of Claim 1, operatively linked to regulatory elements allowing transcription and/or expression of the nucleic acid molecule in plant cells.

9. The plant or part of a plant according to Claim 8, wherein said plant is a cereal or an oily plant.

10. The plant or part of a plant according to Claim 8, wherein said plant is selected from the group consisting of maize, rice, sorghum, wheat, barley, rye, rape, pea, *Brassica napus,* sunflower and sugar cane.

11. A protein encoded by a nucleic acid molecule of any one of Claims 1, the sequence of which is SEQ ID N° 2.

12. A method for obtaining a plant having increased seed size, said method comprising the steps consisting of: a) transforming at least a plant cell or plant tissue by means of at least a vector according to claim 6; b) cultivating the cell(s) or plant tissue thus transformed so as to generate a plant containing in its genome at least an expression cassette according to any one of claims 2 to 5, whereby said plant has increased seed size.

13. A method for increasing plant growth rate, said method comprising the steps consisting of: a) transforming at least a plant cell or plant tissue by means of at least a vector according to claim 6; b) cultivating the cell(s) or plant tissue thus transformed so as to generate a plant containing in its genome at least an expression cassette according to any one of claims 2 to 5.


SEQUENCE LISTING

<110>  GENOPLANTE-VALOR
       UNIVERSITÄT HAMBURG

<120>  Gene involved in the development of the seed

<130>  BRV 24 - EP PRIO

<160>  9

<170>  PatentIn version 3.3

<210>  1
<211>  489
<212>  PRT
<213>  Zea mays

<400>  1

Met Gly Gly Phe His Phe His His Thr Trp Phe Lys Ala Arg Lys Val
1               5                   10                  15

Phe Val Arg Met Leu Ser Asn Gly Gly Gly Ala Ser Met Ala Cys Asp
            20                  25                  30

Ile Ser Val Asp Ala Ser Asp Leu Ser Lys Arg Leu Cys Lys Leu Ile
        35                  40                  45

Ile Ser Cys Arg Lys Ala Ser Ala Leu Glu His Glu Leu Asp His Ser
    50                  55                  60

Gly Val Arg Val Thr Pro Glu Ile Ala Glu Ser Val Leu Lys His Leu
65                  70                  75                  80

Asp Asn Ala Gly Met Leu Ala Tyr Arg Phe Phe Glu Trp Ala Arg Lys
                85                  90                  95

Gln Lys Arg Gly Val Cys Ala His Thr Val Arg Ser Tyr His Thr Val
            100                 105                 110

Val Thr Ser Leu Ala Lys Ile Arg Gln Tyr Gln Leu Met Trp Asp Val
        115                 120                 125

Val Ala Ile Met Arg Lys Glu Gly Val Ala Asn Val Glu Thr Phe Gly
    130                 135                 140

Ile Ile Met Arg Lys Tyr Ala Arg Ala Gln Lys Leu Asp Glu Ala Val
145                 150                 155                 160

Tyr Thr Phe Asn Val Met Glu Lys Tyr Gly Val Ala His Asn Leu Ala

                165                 170                 175

Ala Phe Asn Ser Leu Leu Ser Ala Leu Cys Lys Ser Lys Asn Val Arg
            180                 185                 190

Lys Ala Gln Glu Ile Phe Asp Lys Met Asn Asn Arg Phe Ser Pro Asp
        195                 200                 205

Ala Lys Thr Tyr Ser Ile Leu Leu Glu Gly Trp Gly Arg Ala Pro Ser
    210                 215                 220

Leu Pro Lys Met Arg Glu Val Tyr Ser Asp Met Leu Ala Ala Gly Cys
225                 230                 235                 240

Gln Pro Asp Ile Val Thr Tyr Gly Ile Met Val Asp Ala Leu Cys Lys
                245                 250                 255

Thr Gly Arg Val Glu Glu Ala Val Cys Val Val Gln Asp Met Ser Ser
            260                 265                 270

Arg Gly Cys Gln Pro Thr Thr Phe Ile Tyr Ser Ile Leu Val His Thr
        275                 280                 285

Tyr Gly Val Asp Met Arg Ile Glu Asp Ala Val Ala Ala Phe Leu Asp
    290                 295                 300

Met Glu Lys Asp Gly Ile Met Pro Asp Val Val Val Tyr Asn Ala Leu
305                 310                 315                 320

Ile Thr Ala Phe Cys Lys Val Lys Lys Phe Glu Asn Ala Phe Arg Val
                325                 330                 335

Met Asp Asp Met Gln Gly His Gly Ile Ser Pro Asn Ser Arg Thr Trp
            340                 345                 350

Asn Ile Ile Leu Asn Thr Leu Ile Ser Leu Gly Lys Asp Asp Glu Ala
        355                 360                 365

Tyr Arg Val Phe Arg Ser Met Ile Lys Arg Cys Lys Pro Asp Ser Asp
    370                 375                 380

Thr Tyr Thr Met Met Ile Lys Met Phe Cys Glu Asn Asp Lys Ile Glu
385                 390                 395                 400

Met Ala Leu Lys Val Trp Lys Tyr Met Arg Leu Lys Gln Phe Leu Pro
                405                 410                 415

Ser Met His Thr Phe Ser Val Leu Ile Asn Gly Leu Cys Asp Lys Gly
            420                 425                 430

Glu Val Ser Gln Ala Cys Val Leu Leu Glu Asp Met Ile Glu Lys Gly
        435                 440                 445

Ile Arg Pro Pro Gly Ser Thr Phe Gly Lys Leu Arg Gln Leu Leu Leu
    450                 455                 460

Lys Glu Gly Arg Lys Asp Val Leu Asp Phe Leu Val Glu Lys Met Lys
465                 470                 475                 480

Ile Leu Ala Gln Glu Pro Leu Phe Asp
                485

<210>  2
<211>  1467
<212>  DNA
<213>  Zea mays

<400>  2
atgggtggct tccacttcca ccacacctgg ttcaaggcac gcaaggtgtt tgtaagaatg      60

cttagcaatg gaggcggtgc cagcatggcc tgtgatatct cggtggacgc ctcggacctc     120

tccaagcgcc tctgcaagct catcatctcc tgccggaagg catccgccct cgagcacgag     180

ctcgaccata gcggcgtccg cgttacgccg gagatcgcgg agagcgtcct caagcacctc     240

gacaacgctg gtatgctcgc gtaccgcttc ttcgagtggg cgcgcaagca gaagcgtggg     300

gtgtgtgctc ataccgtgcg ttcctaccac acggtggtta cgtccctagc caagatcagg     360

cagtaccaac tcatgtggga cgtcgtggcc atcatgcgca aggagggtgt ggccaatgtt     420

gagacgttcg ggatcatcat gcgcaaatat gccagggcac agaagttgga tgaggcagtt     480

tacacgttca acgtcatgga gaaatatggt gttgcccaca acctcgctgc cttcaacagc     540

ttgctcagtg cactatgcaa atctaagaac gtgcgcaagg cgcaggagat ctttgataag     600

atgaataatc ggttcagtcc tgatgccaag acctatagta tcttgcttga gggttggggg     660

agagcgccca gcctcccaaa gatgcgagag gtttacagtg atatgcttgc cgctggttgc     720

cagcctgaca ttgtcacata tggtatcatg gtcgatgcac tctgcaagac aggccgagtt     780

gaagaggctg tctgtgtcgt gcaggacatg agctctaggg gatgccaacc gacaaccttc     840

atatacagca tcttagtgca tacttatgga gttgatatga ggattgagga tgctgttgca     900

gcatttttgg atatggagaa ggatgggatt atgcctgatg ttgttgtgta caatgcgctc     960

atcactgcct tctgcaaagt gaagaagttt gagaatgcct tcagagtcat ggatgacatg    1020

caaggccatg gaatctcccc aaattcaagg acctggaaca tcatcctaaa tactctgatt 1080

agccttggaa aggatgatga ggcatatagg gtcttccgca gcatgattaa gcgctgcaaa 1140

ccagactctg atacttacac catgatgata aagatgttct gtgagaatga taagatagag 1200

atggcactga aggtatggaa gtatatgcga ttgaagcagt tcttgccgag catgcacaca 1260

ttctctgtgc tgatcaatgg tctctgtgac aagggtgaag ttagccaagc ttgtgtcctg 1320

cttgaagata tgatcgagaa gggcattaga cctcctggtt caacttttgg caagctgagg 1380

cagctacttt taaaagaagg gaggaaggat gtgcttgact ttcttgtcga gaaaatgaag 1440

atcctcgctc aagaaccttt gtttgat 1467

<210> 3
<211> 2460
<212> DNA
<213> Zea mays

<400> 3
tcgaagtggg ctgtgggtcg cgtcagctcc tgaggcaagt agtctagtga cttcgtcctc 60

ttgaccggac ttcgccgccg cgccgcgccg cgcagagtac tccggcgacg cggccatttt 120

ttgggtcgag ccgagcaccg gaatctttgc cctttttctc gctttctgag gttttagctc 180

acgagcattg ggccttacga tttctcatca aacctatgtt ggcccaatca gttcaccagg 240

cttaaagccc atatgcttct gccttcactg atggcccacg ttatcacttc ggcgagttca 300

ctccacgata acggaccgga tcgcacttga cgatcaaatc gaatcgcacc tattcctctc 360

tttggcggta gccgcgtagc gcccgctcaa tcgccgccga ccacggcgga gaaattgcgg 420

cttcacctcg cctgtgtgtc ctgattacgc gaggactcga gcggctaagc ttagcagcga 480

tctcggtctc agcgaggtcg gtgggtgccg gaagagaaat gggtggcttc cacttccacc 540

acacctggtt caaggcacgc aaggtgtttg taagaatgct tagcaatgga ggcggtgcca 600

gcatggcctg tgatatctcg gtggacgcct cggacctctc caagcgcctc tgcaagctca 660

tcatctcctg ccggaaggca tccgccctcg agcacgagct cgaccatagc ggcgtccgcg 720

ttacgccgga gatcgcggag agcgtcctca agcacctcga caacgctggt atgctcgcgt 780

accgcttctt cgagtgggcg cgcaagcaga agcgtggggt gtgtgctcat accgtgcgtt 840

cctaccacac ggtggttacg tccctagcca agatcaggca gtaccaactc atgtgggacg 900

tcgtggccat catgcgcaag gagggtgtgg ccaatgttga gacgttcggg atcatcatgc 960

gcaaatatgc cagggcacag aagttggatg aggcagttta cacgttcaac gtcatggaga 1020

aatatggtgt tgcccacaac ctcgctgcct tcaacagctt gctcagtgca ctatgcaaat 1080

ctaagaacgt gcgcaaggcg caggagatct ttgataagat gaataatcgg ttcagtcctg 1140

atgccaagac ctatagtatc ttgcttgagg gttgggggag agcgcccagc ctcccaaaga 1200

tgcgagaggt ttacagtgat atgcttgccg ctggttgcca gcctgacatt gtcacatatg 1260

gtatcatggt cgatgcactc tgcaagacag gccgagttga agaggctgtc tgtgtcgtgc 1320

aggacatgag ctctagggga tgccaaccga caaccttcat atacagcatc ttagtgcata 1380

cttatggagt tgatatgagg attgaggatg ctgttgcagc atttttggat atggagaagg 1440

atgggattat gcctgatgtt gttgtgtaca atgcgctcat cactgccttc tgcaaagtga 1500

agaagtttga gaatgccttc agagtcatgg atgacatgca aggccatgga atctccccaa 1560

attcaaggac ctggaacatc atcctaaata ctctgattag ccttggaaag gatgatgagg 1620

catatagggt cttccgcagc atgattaagc gctgcaaacc agactctgat acttacacca 1680

tgatgataaa gatgttctgt gagaatgata agatagagat ggcactgaag gtatggaagt 1740

atatgcgatt gaagcagttc ttgccgagca tgcacacatt ctctgtgctg atcaatggtc 1800

tctgtgacaa gggtgaagtt agccaagctt gtgtcctgct tgaagatatg atcgagaagg 1860

gcattagacc tcctggttca acttttggca agctgaggca gctactttta aaagaaggga 1920

ggaaggatgt gcttgacttt cttgtcgaga aaatgaagat cctcgctcaa gaacctttgt 1980

ttgattgacc cttcacttct ccattggtat gaatgcttac tcctgtcatc tatatggata 2040

cacgtggtta atatgggatg ggtagatgct caaaatatct gctcttattt cttcattctt 2100

taatttgcag tattatttac tcccccgccc aaaatataat tcatttagat taaatatgca 2160

ttcattaatt aatctatgaa tgtagtttat aaattatatg tatgtccatg ttcattatca 2220

tttattcaaa tgtggacgaa aaaagaaggc taaaaagaac tatattttgg aatggaggga 2280

gtattattct atatctgagt ttctgttgct gatatactct tcatggttca tgtgccactc 2340

cagctgctga gaatatcagt tatcatttag gctttatgaa taaatgttcc cagttaagga 2400

ttgctttggt gacccaggat catagaagga ttggaagggg attgaggtgt tttttagtca 2460

<210> 4
<211> 487
<212> PRT
<213> Sorghum bicolor

<400> 4

Met Gly Asp Leu His His Thr Trp Leu Lys Ala Arg Lys Val Phe Val
1               5                   10                  15

Arg Met Leu Ser Ser Gly Gly Gly Ala Ser Val Gly Cys Asp Thr Leu
            20                  25                  30

Leu Asp Ala Ser Asp Leu Ala Lys Arg Leu Cys Lys Leu Ile Ile Ser
        35                  40                  45

Cys Arg Lys Ala Ser Ala Val Glu His Glu Leu Asp His Ser Gly Val
    50                  55                  60

Arg Val Thr Pro Glu Val Ala Glu Arg Val Leu Glu Arg Leu Asp Asn
65                  70                  75                  80

Ala Gly Met Leu Ala Tyr Arg Phe Phe Glu Trp Ala Arg Arg Gln Lys
                85                  90                  95

Arg Gly Gly Cys Ala His Thr Val Arg Ser Tyr His Thr Val Val Ala
            100                 105                 110

Ser Leu Ala Lys Ile Arg Gln Tyr Gln Leu Met Trp Asp Val Val Ala
        115                 120                 125

Val Met Arg Lys Glu Gly Val Ala Asn Val Glu Thr Phe Ser Ile Ile
    130                 135                 140

Met Arg Lys Tyr Ala Arg Ala Gln Lys Phe Asp Glu Ala Val Tyr Thr
145                 150                 155                 160

Phe Asn Ile Met Glu Arg Tyr Gly Val Ala His Asn Leu Ala Ala Phe
                165                 170                 175

Asn Ser Leu Leu Gly Ala Leu Cys Lys Ser Lys Asn Val Arg Lys Ala
            180                 185                 190

Gln Glu Ile Phe Asp Lys Met Asn Asn Arg Phe Ser Pro Asp Ala Lys
        195                 200                 205

Thr Tyr Ser Ile Leu Leu Glu Gly Trp Gly Arg Ala Pro Asn Leu Pro
    210                 215                 220

Lys Met Arg Glu Val Tyr Ser Asp Met Leu Ala Ala Gly Cys Gln Pro
225                 230                 235                 240

Asp Ile Val Thr Tyr Gly Ile Met Val Asp Ala Leu Cys Lys Thr Gly
                245                 250                 255

Arg Val Glu Glu Ala Val Cys Val Val Gln Asp Met Ser Ser Arg Gly
            260                 265                 270

Cys Gln Pro Thr Thr Phe Ile Tyr Ser Val Leu Val His Thr Tyr Gly

275                 280                 285

Val Asp Met Arg Ile Glu Asp Ala Val Ala Thr Phe Leu Asp Met Glu
    290                 295                 300

Lys Asp Gly Ile Val Pro Asp Val Val Val Tyr Asn Ala Leu Val Thr
305                 310                 315                 320

Ala Phe Cys Lys Val Lys Lys Phe Asp Asn Ala Phe Arg Val Met Asp
                325                 330                 335

Asp Met Glu Gly His Gly Ile Ser Pro Asn Ser Arg Thr Trp Asn Ile
            340                 345                 350

Ile Leu Asn Thr Leu Ile Ser Leu Gly Lys Asp Asp Glu Ala Tyr Arg
        355                 360                 365

Val Phe Arg Asn Met Ile Lys Arg Cys Lys Pro Asp Ser Asp Thr Tyr
    370                 375                 380

Thr Met Met Ile Lys Met Phe Cys Glu Asn Asp Lys Ile Glu Met Ala
385                 390                 395                 400

Leu Lys Val Trp Lys Tyr Met Arg Leu Lys Gln Phe Leu Pro Ser Met
                405                 410                 415

His Thr Phe Ser Val Leu Ile Asn Gly Leu Cys Asp Lys Gly Glu Val
            420                 425                 430

Ser Gln Ala Cys Val Leu Leu Glu Asp Met Ile Glu Lys Gly Ile Arg
        435                 440                 445

Pro Pro Gly Ser Thr Phe Gly Lys Leu Arg Gln Leu Leu Leu Lys Glu
    450                 455                 460

Gly Arg Lys Asp Val Leu Asp Phe Leu Val Glu Lys Met Lys Met Leu
465                 470                 475                 480

Ile Gln Glu Pro Leu Phe Asp
                485

<210>  5
<211>  484
<212>  PRT
<213>  Oryza sativa

<400>  5

Met Gly Gly Val Arg Pro Thr Leu Phe Asp Ala Arg Lys Val Phe Val
1               5                   10                  15

Arg Met Leu Ser Ser Gly Val Gly Gly Gly Asp Ala Leu Val Asp Thr
            20                  25                  30

Ala Asp Pro Ala Lys Arg Leu Phe Lys Leu Ile Ile Ser Cys Arg Lys
        35                  40                  45

Ala Ser Ala Val Glu His Glu Leu Asp His Ser Gly Val Arg Val Thr
    50                  55                  60

Pro Asp Val Ala Glu Arg Val Leu Glu Arg Leu Asp Asn Ala Gly Met
65                  70                  75                  80

Leu Ala Tyr Arg Phe Phe Glu Trp Ala Arg Arg Gln Lys Arg Gly Gly
                85                  90                  95

Cys Ala His Thr Val Arg Ser Tyr His Thr Val Val Ala Ser Leu Ala
            100                 105                 110

Lys Ile Arg Gln Tyr Gln Leu Met Trp Asp Val Val Ala Val Met Arg
        115                 120                 125

Arg Glu Gly Ala Val Asn Val Glu Thr Phe Gly Ile Ile Met Arg Lys
    130                 135                 140

Tyr Ala Arg Ala Gln Lys Val Asp Glu Ala Val Tyr Thr Phe Asn Val
145                 150                 155                 160

Met Glu Lys Tyr Gly Val Val Pro Asn Leu Ala Ala Phe Asn Ser Leu
                165                 170                 175

Leu Gly Ala Leu Cys Lys Ser Lys Asn Val Arg Lys Ala Gln Glu Ile
            180                 185                 190

Phe Asp Lys Met Asn Ser Arg Phe Ser Pro Asp Ala Lys Thr Tyr Ser
        195                 200                 205

Ile Leu Leu Glu Gly Trp Gly Arg Ala Pro Asn Leu Pro Lys Met Arg
    210                 215                 220

Glu Val Tyr Ser Glu Met Leu Asp Ala Gly Cys Glu Pro Asp Ile Val
225                 230                 235                 240

Thr Tyr Gly Ile Met Val Asp Ser Leu Cys Lys Thr Gly Arg Val Glu
                245                 250                 255

Glu Ala Val Arg Val Val Gln Asp Met Thr Ser Arg Gly Cys Gln Pro
            260                 265                 270

Thr Thr Tyr Ile Tyr Ser Val Leu Val His Thr Tyr Gly Val Glu Met
        275                 280                 285

Arg Ile Glu Asp Ala Val Ala Thr Phe Leu Asp Met Glu Lys Asp Gly
    290                 295                 300

Ile Val Pro Asp Ile Val Val Tyr Asn Ala Leu Val Ser Ala Phe Cys
305                 310                 315                 320

Lys Ala Lys Lys Phe Glu Asn Ala Phe Arg Val Leu Asn Asp Met Glu
                325                 330                 335

Gly His Gly Ile Thr Thr Asn Ser Arg Thr Trp Asn Ile Ile Leu Asn
            340                 345                 350

His Leu Ile Ser Leu Gly Arg Asp Asp Glu Ala Tyr Lys Val Phe Arg
        355                 360                 365

Arg Met Ile Lys Cys Cys Gln Pro Asp Cys Asp Thr Tyr Thr Met Met
    370                 375                 380

Ile Lys Met Phe Cys Glu Asn Asp Lys Val Glu Met Ala Leu Lys Val
385                 390                 395                 400

Trp Lys Tyr Met Arg Leu Lys Gln Phe Leu Pro Ser Met His Thr Phe
                405                 410                 415

Ser Val Leu Ile Asn Gly Leu Cys Asp Lys Arg Glu Val Ser Gln Ala
            420                 425                 430

Cys Val Leu Leu Glu Asp Met Ile Glu Lys Gly Ile Arg Pro Pro Gly
        435                 440                 445

Ser Thr Phe Gly Lys Leu Arg Gln Leu Leu Leu Lys Glu Gly Arg Lys
    450                 455                 460

Asp Val Leu Asp Phe Leu Val Glu Lys Met Asn Ile Leu Ile Gln Glu
465                 470                 475                 480

Pro Leu Phe Asp

<210>  6
<211>  481
<212>  PRT
<213>  Arabidopsis thaliana

<400>  6

Met Thr Ile Arg Ile Ile Asn Arg Gln Ser Arg Phe Cys Cys Lys Ser
1               5                   10                  15

Phe Leu Ser Ala Arg Leu Tyr Ser Ser Ser Glu Gln Val Arg Asp Val
            20                  25                  30

Ala Asp Val Ala Lys Asn Ile Ser Lys Val Leu Met Ser Ser Pro Gln
        35                  40                  45

Leu Val Leu Asp Ser Ala Leu Asp Gln Ser Gly Leu Arg Val Ser Gln
    50                  55                  60

Glu Val Val Glu Asp Val Leu Asn Arg Phe Arg Asn Ala Gly Leu Leu
65                  70                  75                  80

Thr Tyr Arg Phe Phe Gln Trp Ser Glu Lys Gln Arg His Tyr Glu His
                85                  90                  95

Ser Val Arg Ala Tyr His Met Met Ile Glu Ser Thr Ala Lys Ile Arg
            100                 105                 110

Gln Tyr Lys Leu Met Trp Asp Leu Ile Asn Ala Met Arg Lys Lys Lys
        115                 120                 125

Met Leu Asn Val Glu Thr Phe Cys Ile Val Met Arg Lys Tyr Ala Arg
    130                 135                 140

Ala Gln Lys Val Asp Glu Ala Ile Tyr Ala Phe Asn Val Met Glu Lys
145                 150                 155                 160

Tyr Asp Leu Pro Pro Asn Leu Val Ala Phe Asn Gly Leu Leu Ser Ala
                165                 170                 175

Leu Cys Lys Ser Lys Asn Val Arg Lys Ala Gln Glu Val Phe Glu Asn
            180                 185                 190

Met Arg Asp Arg Phe Thr Pro Asp Ser Lys Thr Tyr Ser Ile Leu Leu
        195                 200                 205

Glu Gly Trp Gly Lys Glu Pro Asn Leu Pro Lys Ala Arg Glu Val Phe
    210                 215                 220

Arg Glu Met Ile Asp Ala Gly Cys His Pro Asp Ile Val Thr Tyr Ser
225                 230                 235                 240

Ile Met Val Asp Ile Leu Cys Lys Ala Gly Arg Val Asp Glu Ala Leu
                245                 250                 255

Gly Ile Val Arg Ser Met Asp Pro Ser Ile Cys Lys Pro Thr Thr Phe
            260                 265                 270

Ile Tyr Ser Val Leu Val His Thr Tyr Gly Thr Glu Asn Arg Leu Glu
        275                 280                 285

Glu Ala Val Asp Thr Phe Leu Glu Met Glu Arg Ser Gly Met Lys Ala
    290                 295                 300

Asp Val Ala Val Phe Asn Ser Leu Ile Gly Ala Phe Cys Lys Ala Asn
305                 310                 315                 320

Arg Met Lys Asn Val Tyr Arg Val Leu Lys Glu Met Lys Ser Lys Gly
                325                 330                 335

Val Thr Pro Asn Ser Lys Ser Cys Asn Ile Ile Leu Arg His Leu Ile
            340                 345                 350

Glu Arg Gly Glu Lys Asp Glu Ala Phe Asp Val Phe Arg Lys Met Ile
        355                 360                 365

Lys Val Cys Glu Pro Asp Ala Asp Thr Tyr Thr Met Val Ile Lys Met
    370                 375                 380

Phe Cys Glu Lys Lys Glu Met Glu Thr Ala Asp Lys Val Trp Lys Tyr
385                 390                 395                 400

Met Arg Lys Lys Gly Val Phe Pro Ser Met His Thr Phe Ser Val Leu
                405                 410                 415

Ile Asn Gly Leu Cys Glu Glu Arg Thr Thr Gln Lys Ala Cys Val Leu
            420                 425                 430

Leu Glu Glu Met Ile Glu Met Gly Ile Arg Pro Ser Gly Val Thr Phe
        435                 440                 445

Gly Arg Leu Arg Gln Leu Leu Ile Lys Glu Glu Arg Glu Asp Val Leu
    450                 455                 460

Lys Phe Leu Asn Glu Lys Met Asn Val Leu Val Asn Glu Pro Leu Cys
465                 470                 475                 480

Asp

<210>  7
<211>  25
<212>  DNA
<213>  Artificial

<220>
<223>  Primer OmuA

<400>  7
cttcgtccat aatggcaatt atctc                                          25

<210>  8
<211>  20
<212>  DNA
<213>  Artificial

<220>
<223>  Primer PPR_R03

<400>  8
acaatgtcag gctggcaacc                                                20

<210>  9
<211>  21
<212>  DNA
<213>  Artificial

<220>
<223>  Primer PPR_F03

<400>  9
gaaatgggtg gcttccactt c                                              21